# EUROPEAN PATENT APPLICATION

(11) **EP 3 399 169 A1**
(43) Date of publication of application: **07.11.2018**
(21) Application number: 16889188.5
(22) Date of filing: 30.12.2016
(51) Int. Cl.: F02C 6/04, C07D 307/50, C07C 1/04, B01J 27/13, C10L 5/44, C10L 5/46

(54) **SYSTEM FOR PRODUCING ENERGY FROM SEWAGE AND DOMESTIC AND AGRICULTURAL WASTE BY INTEGRATING FOUR APPARATUSES**

(30) Priority: 30.12.2015 GC L201530684
(71) Applicant: Alsalahi, Bader Mousa Ahmed, Makkah 7060-24267 (SA); Abu-Elyazeed, Osayed Sayed M., Asharqya 11718 (EG)
(72) Inventor: Alsalahi, Bader Mousa Ahmed, Makkah 7060-24267 (SA); Abu-Elyazeed, Osayed Sayed M., Asharqya 11718 (EG)
(74) Representative: Postiglione, Ferruccio
(86) International application number: PCT/IB2016/058125
(87) International publication number: WO 2017/134505

(57) **Abstract**

**Digester 1** contains the sewage present in diluted acid and temperature of 35-45 °C of anaerobic digestion and the production of biogas after a digestion period of 15-20 days, which can be used as fuel for **power generator 3**. **Power generator 3** produces electrical power being the **first product** of the device, and also produces exhaust that is used to supply **gas generator 2** with heat and gases with oxygen. **Gas generator 2** is supplied with household waste and the rest of digestion process, and the rest of the agricultural waste treatment process which is obtained from **extractor 7**. **Gas generator 2** produces active coal and ashes as **second and third products** of this invention, and also produces syngas. Syngas is purified in chambers surrounding **digester 1** to be heated, as such chambers contain dolomite, eggshell, charcoal, ashes, and sand to separate tar being the **fourth product** from this device. Meanwhile, purified syngas is cooled in two phases; the first phase is done in **primary treatment tank 9** to convert the agricultural cellulosic wastes into glucose during the hydrolysis for 4 hours in diluted hydrochloric acid solution at 3%. The second phase of syngas cooling is done in **radiator 4**. About half the syngas after cooling is supplied to **power generator 3**, while the rest is compressed to 25 bar, heated to 300 °C through **compressor 11** and then moved to **reactor 6** in the presence of silicon carbide -calcium - cobalt as a catalyst for the conversion of syngas into biodiesel being the **fifth product** of this device. Furthermore, the glucose produced during the hydrolysis of agricultural cellulosic waste is dissolved by the addition of DMSO to the glucose in **primary treatment tank 9** and stirring the mixture for an hour in the presence of chromic chloride to convert glucose into fructose. This mixture comprises of DMSO, glucose, fructose and chromic chloride. The unconverted biomass is pumped through **primary treatment tank 9** to **reactor 6** by **pump 10** for the production of HMF being the **sixth product** through heating mixture at 120°C or 250°C for two hours or fifteen minutes, respectively, by the use of the above-mentioned compressed syngas. The resulting product, which is HMF, DMSO, chromic chloride and unconverted biomass, is first poured into **extractor 7** to separate the remaining biomass and then the residue is supplied first to **gas generator 2**, and second to **purification tank 8** filled with active coal to adsorb the solvent DMSO and extract the purified HMF being the **sixth product** of this invention. **Purification tank 8** is heated to 192 °C to evaporate the solvent DMSO, and then such steam is intensified in **radiator 5**.

## Description

### Technical Field:

This invention relates to an integrated process of generating diverse types of energy using different types of wastes.

### Prior Art:

Presently, the energy crisis is well-known to be caused by the lack of energy resources, such as petroleum oil, due to the decrease in production and increase in consumption. Additionally, the environmental impact of oil, gas and coal combustion causes a crisis known as the greenhouse effect; let alone the fact that the usual methods of disposing of wastes, such as sewage, household and agricultural wastes intensify this environmental catastrophe.

It is noteworthy that electricity is generated using thermal devices without hot exhausts estimated at 30% of the thermal content of the used fuel. Accordingly, these devices, such as diesel motors and gas turbines, can play a significant role in overcoming the energy and environmental crisis.

Waste, also known as "biomass", can also be classified into two types; the first is recyclable waste, such as paper, plastic material and metal that are reprocessed to produce useful products. The second type of waste is called unacceptable waste, such as sewage, food residues and agricultural wastes and is divided into volatile and nonvolatile substances and ashes. This type of biomass can be used as an extraordinary source of energy using one or more of the three following methods: anaerobic digestion, thermal conversion to gases and thermochemical conversion. As such, any of the abovementioned methods can be either endothermic, such as anaerobic digestion and thermochemical conversion or exothermic such as thermal conversion to gases. In commonly known devices, any of the abovementioned methods can be used separately without any linkage among them or any linkage to power generation devices.

However, there is a serious lack of integration between biomass conversion devices and power generation devices. In addition, there is a lack of knowledge and technology in the field of thermochemical conversion of agricultural waste, as is the case in the field of purification of different products such as synthetic gases (syngas) and hydroxymethylfurfural (HMF), which has not been resolved until now.

### Disclosure of the Invention:

The present invention does not only aim to achieve integration among four devices by using the exhausts or outputs of any device to feed one or a plurality of the other devices, but also aims at purifying HMF and syngas using natural and inexpensive materials. The first device is an electrical power generator using a diesel motor, gas turbine or steam cycle as a primary generator with hot gases as exhausts. The second device is an anaerobic digester for producing biogas with a carbon-rich material as an exhaust of the endothermic process. The third device is a system for converting biomass to gases for production of syngas, biodiesel, tar, charcoal and ashes with heat as an exhaust of the exothermic process. The fourth and last device is a thermochemical converter for producing HMF with non-convertible solids as an exhaust of the endothermic process.

It is obvious from the foregoing that this invention can achieve integration among the four abovementioned devices. In addition, this invention requires complex connections and control system for all of the four devices in order to ensure integration among them and also requires actual data resulting from practical experiences in different types of conditions such as flow rate, reaction time, temperature, pressure, catalyst type and pH.

The above separate devices are low-energy density devices (generated energy/consumed energy) because of the use of external energy resources (heat and electricity, etc.) as is the case in exhausts (substances and heat, etc.). The present invention is known as a high-energy density device consisting of four zero-emission parts due to the use of exhausts as energy or a substance from any part as a source of the other parts of the device.

Accordingly, the inputs to the first part, electrical power generator, are air and fuel (the biogas from the second part, the anaerobic digester, a percentage of the syngas produced from the third part of the invention and the gas generator from biomass). Furthermore, the outputs of this first part are electrical power as a final product in addition to high-temperature exhaust gases as a source of heat and partially oxygen-containing gases for the third part of the invention for completing the conversion to gases.

The inputs to the second part of this invention, anaerobic digester, are sewage (wastes), heat (generated from the syngas produced from the third part, the system of conversion of biomass to gases during the process of purifying the gases), anaerobic bacteria (from soil) and an acidic solution added to adjust the pH of the solution in the digestion area, while the outputs from this part are biogas (used as a fuel of the power generator, first part of the device) and carbon-rich materials (used as a fertilizer in unpolluted drainage or a source of biomass of the third part of the invention).

As such, inputs to the third part of this device, the system of converting biomass to gases are biomass (household wastes, carbon-rich materials produced from the second part of the device and solids produced from the fourth part of the invention, thermochemical conversion of agricultural wastes), oxygen-containing gases (generated from the exhaust of the first part of the device, power generator, and the air), a heat source (from the hot exhaust gases generated from the first); while the outputs from the third part are heat (used to increase the temperature of the second part and the hydrolysis processing of agricultural wastes in the fourth part of the invention), syngas, a hydrogen-rich gas purified in chambers around the digester containing dolomite, eggshell, charcoal, ashes and sands as catalysts (about half of these gases are used as fuel for the first part, while the other half is converted into biodiesel during the Fischer-Tropsch process of compressed syngas as a final second product of the invention with silicon carbide, cobalt and calcium as catalysts with shelf life of five years). Tar is a final third product produced during the syngas-cooling process before the syngas compression and charcoal is the final fourth product produced from converting the biomass to gases, as is the case in ashes, the final fifth product.

The fourth part of the invention is a thermochemical device used to treat agricultural wastes and comprises three phases. The first stage is a hydrolysis process that represents primary treatment of agricultural waste as cellulose materials, and inputs are water (used for hydrolysis of these wastes by converting cellulose to glucose with hydrochloric acid of a volume of 3%) and heat (generated from the third part during syngas cooling and before syngas compression to meet the requirements for Fischer-Tropsch process). The second stage is the dehydration process applied as a final treatment of glucose and converting it to fructose and then to hydroxymethylfurfural (HMF). The inputs of the final sixth product of the invention are heat (generated from the third part during the Fischer-Tropsch process of hot and compressed syngas, with DMSO as solvent and chromium chloride as catalyst). The third stage is a process for purifying the HMF produced from the previous stage and recovering DMSO as a reusable solvent (using charcoal to elute DMSO, as a polarity material, and thus collecting HMF, then the coal-heating process starts to evaporate eluted DMSO and vapor is condensed to recover DMSO). The sixth product of the fourth part is HMF and the remaining non-cellulose solids (used as a source of biomass for the third part of this device, the system of conversion of biomass to gases).

### Description of Drawings & Best Condition for Invention Operation

The invention may be understood and confined to the practical effect. As a detailed example of the invention, the invention will now be described with accompanying drawings, which are:
Figure (1) is a detailed example of the device consisting of four integrated parts for the production of electricity, biodiesel, HMF, charcoal, tar, ashes from waste (sewage, household waste, agricultural waste) built on the invention.
Figure (2) indicates the unique design of digester with purification chamber of syngas.
Figure (3) is a scheme of material flow from and to each component of the integrated device.

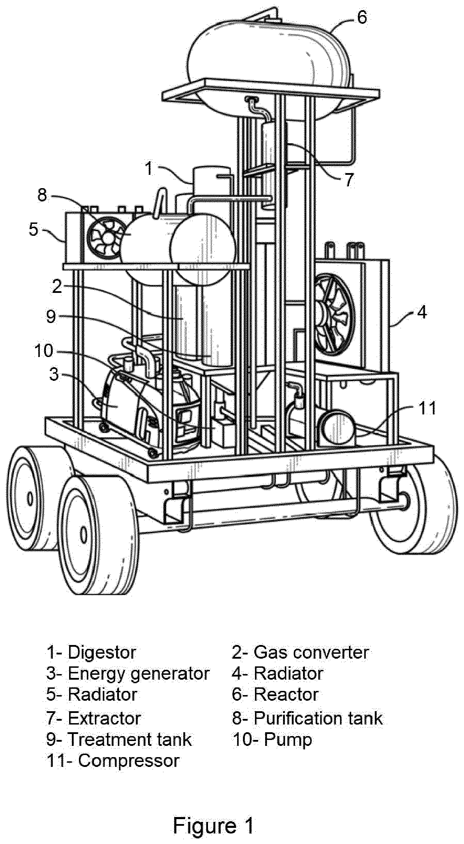

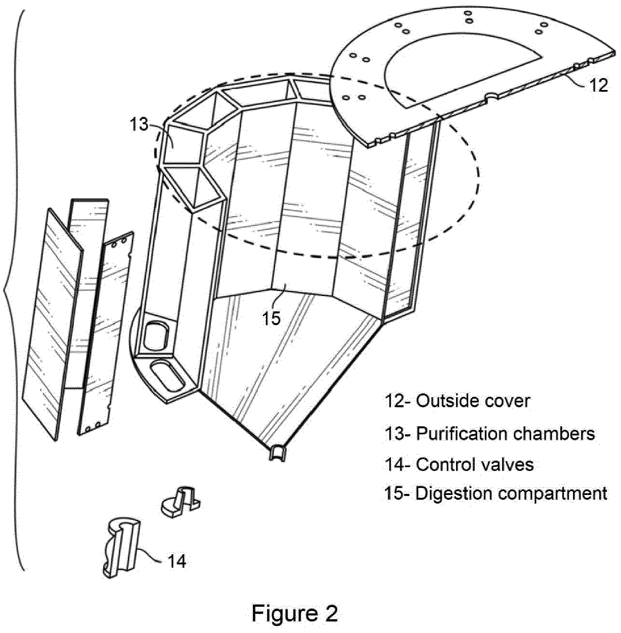

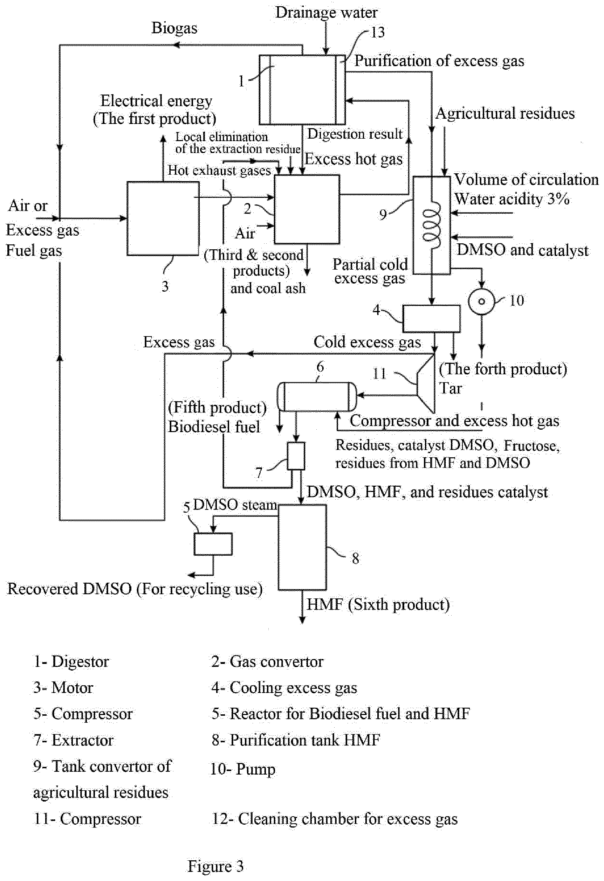

**Digester 1** contains the sewage within circumstances of diluted acid and temperature of 35-45 °C of anaerobic digestion and the production of biogas after a digestion period of 15-20 days, which can be used as fuel for **power generator 3**. This **power generator 3** produces electrical power being the **first product** of the device, and also produces exhaust that is used to provide **gas generator 2** with heat and gases with oxygen as indicated in Figure 1. **Gas generator 2** is provided with household waste and the rest of the digestion process, as well as the rest of the agricultural waste treatment process and what is obtained from **extractor 7**. **Gas generator 2** produces active coal and ashes representing the **second and third products** of this invention, and also produces syngas. Syngas is purified in chambers surrounding **digester 1** for heating purposes, as such chambers contain the dolomite, eggshell, charcoal, ashes, sand to separate tar being the **fourth product** of this device. Purified syngas is cooled in two phases, the first of which is done in **primary treatment tank 9** to convert the agricultural cellulosic waste into glucose during the hydrolysis for four hours in diluted hydrochloric acid solution at 3%. The second phase of syngas cooling is done in **radiator 4**. About half of the syngas after cooling is provided to **power generator 3**, while the rest is compressed to 25 bar, heated to 300 °C through **compressor 11** and then moved to **reactor 6** in presence of silicon carbide -calcium - cobalt as a catalyst for the conversion of syngas into biodiesel being the **fifth product** of this device. The glucose produced during the hydrolysis of agricultural cellulosic waste is melted by adding DMSO to this glucose in **primary treatment tank 9** and mixing this mixture for an hour in presence of chromic chloride to convert this glucose into fructose. This mixture is composed of DMSO, glucose, fructose and chromic chloride. The unconverted biomass is pumped through **primary treatment tank 9** through **pump 10** to **reactor 6** for the production of HMF being the **sixth product** by heating this mixture at 120 °C or 250 °C for two hours or fifteen minutes, respectively, through the use of the above-mentioned compressed syngas. The new resulted product, which is HMF, DMSO, chromic chloride and unconverted biomass, is first poured into **extractor 7** to separate the remaining biomass and then supply the residue to **gas generator 2**, and second to **purification tank 8** filled with active coal to adsorb the solvent DMSO and extract the purified HMF being the **sixth product** of this invention.

**Also, purification tank 8** is heated to 192 °C to evaporate the solvent DMSO, then this steam is condensed in **radiator 5**.

With reference to Figure (2), there are 3D details of the unique design of **digester 1**. This design is based on the execution of the principle of downsizing this part. It contains **upper cover 12**, which can be used as cover of **chamber 13** and **digestion area 15**. It is also equipped with holes for refilling with sewage and removal of produced biogas, holes for measurement and security and a tool to flip sewage inside the digester. Chamber 13 is arranged to form **digestion area 15** and is considered as cleaning passages of syngas filled with dolomite, egg shell, active coal, ashes and sand as catalytic agents. Syngas is also used to heat up **digestion area 15**. The syngas, together with the remaining part after digestion, can be removed through **control valves 14**.

Figure (3) depicts the substance flow scheme to confirm different types of methods as previously showed in Figure (1), as follows:
The present device can be divided into four parts; the first which can be called "electrical power producer", which comprises **power generator 3**, while the second part can be called "biogas producer" and comprises **digester 1**. The third part can be called "generator of biodiesel, active coal, tar and ashes" and comprises **purification chamber 13**, **gas generator 2, radiator 4, compressor 11** and **reactor 6**. The fourth part is called "HMF Producer" and contains **primary treatment tank 9, pump 10, reactor 6, extractor 7** and **purification tank 8**.

In any case, with reference to Figure (3), the substances entering into the first part are air and fuel (biogas or syngas from the second and third parts, respectively) for the production of electrical power and hot exhaust gases. The substances entering into the second part are sewage waste and heat (from the third part) and acid solution (to adjust PH of digestion) for the production of biogas as fuel of the first part. The substances entering into the third part are air and exhaust gases for the conversion into gases (from the first part), household waste and silicon carbide, cobalt, calcium as a catalytic agent (for the Fischer-Tropsch process) and the rest of the second and fourth parts for the production of active coal, ashes and biodiesel. The fourth part receives heat (from compressed air in third part) and diluted aqueous acid solution 3%, DMSO as solvent, chromic chloride as a catalytic agent, and active coal to purify produced HMF.

As indicated in Figure (3), for a complete understanding of the entire device, the colored paths shall be followed, as follows:

### Black paths as main entries:

- Sewage of **digester 1**
- Household waste of gas **generator 3**
- Remaining part from **digester 1** to **gas generator 3**
- Remaining part from **extractor 7** to **gas generator 3**
- Agricultural waste of **primary treatment tank 9**

### Main products:

- Electrical power (first product) **from power generator 3**
- Active coal and ashes (second and third product respectively) from gas **generator 3**
- Tar (fourth product) from **radiator 4**
- Biodiesel (fifth product) from **reactor 6**
- HMF (sixth product) from **reactor 6**

### Orange paths of intermediary processes for gases:

- Biogas is produced in **digester 1** and then supplied to **power generator 3** as fuel.
- Hot exhaust gases are produced in **power generator 3** and then supplied to **gas generator 2** to convert the biomass into gases.
- Syngas is generated during the biomass conversion into gases in **gas generator 2**. The gas is then supplied to chamber 13, not only to clean syngas from impurities in the presence of dolomite, eggshell, active coal, ashes and sand as catalytic agents, but also to heat up the digester solution to accelerate the digestion in **digester 1**.
- Hot syngas is moved out from **chamber 13** to be initially cooled during passage through **primary treatment tank 9** through two types of liquids, which are diluted aqueous acid solution 3% and DMSO as solvent in the presence of 1% of mass of chromic chloride as a catalytic agent.
- Initially cooled syngas is removed from **primary treatment tank 9** to be cooled in **radiator 4** to increase tar in syngas to prepare such gases for the compression process or use the same as fuel for **power generator 3**.
- Cooled syngas is supplied to **compressor 11** to be compressed to 25 bar. As a result of such compression, the temperature of syngas increases up to 300 °C to prepare the same for the Fischer-Tropsch process (conversion of syngas into liquid biodiesel in presence of a catalytic agent) in **reactor 6.**
- Compressed syngas is supplied to **reactor 6** at 25 bar and 300 °C in presence of 75% of silicon carbide - 20% of cobalt - 5% of calcium as a catalytic agent for the production of biodiesel.

### Blue paths of intermediary processes for liquids:

- Hot syngas is removed from **chamber 13** for the purpose of heating **primary treatment tank 9** to achieve the hydrolysis of agricultural wastes (conversion of cellulosic substances into glucose) in presence of diluted aqueous acid solution 3% at 180 °C for a period of four to six hours, and the conversion of produced glucose to fructose through strong flipping of such glucose with DMSO as solvent (1 liter of DMSO/0.8 kg of glucose) in presence of 1% of chromic chloride as a catalytic agent at 90 °C for one hour.
- The mixture resulting from the previous process is composed of the rest of waste, DMSO, fructose and a catalytic agent. In addition, such mixture is removed from **primary treatment tank 9** to **reactor 6** by using **pump 10** for the production of HMF through heating this mixture to 120 °C or 250 °C for two hours or fifteen minutes, respectively, in **reactor 6**, through compressed and hot syngas.
- Mixtures previously produced in **reactor 6** are consisting of the rest of the waste, DMSO, HMF and a catalytic agent, which is poured into extractor 7, to separate remaining agricultural wastes and generate solution from DMSO, HMF and a catalytic agent.
- For the purification of HMF, the previous solution is moved to **purification tank 8** which contains active coal to adsorb DMSO and, consequently, remove purified HMF from the bottom of such tank.
- After obtaining DMSO from **purification tank 8** with the presence of active coal and adsorbed DMSO, it can be heated up to 192 °C to evaporate DMSO and then supply resulting steam to **radiator 5** to increase such steam and, as such, recapture DMSO for later use.

## Claims

1. This invention seeks integration among four different devices for the purpose of converting different types of waste to different types of energy resources. This integration is to use exhaust or outputs of any device as an input of one or a plurality of devices. These four devices are as follows: the first part can be called **producer of electric power** which comprises **power generator 3** such as diesel motor, gas turbine or steam cycle as being a main motor of exhaust gases; the second part can be called **producer of biogas** which comprises digester 1; the third part can be called **generator of biodiesel, active coal, tar, and ashes** which comprises **chamber 13** for purification, **gas generator 2, radiator 4, compressor 11,** and **reactor 6**; the fourth part is called **producer of HMF** and comprises primary **treatment tank 9, pump 10, reactor 6, extractor 7 and purification tank 8.**

2. A method according to claim 1, wherein the unique design of **digester 1**, which was formed by assembling and arranging twelve **chambers 13,** is based on achieving integration of this device.

3. The **chambers 13** according to claim 2, can be considered as the purification path of syngas, and are filled with dolomite (in 4 chambers); eggshell (in three chambers); active coal (in two chambers); ashes (in two chambers); and sand (in one chamber) as catalytic agents. This syngas is also used in heating **digestion compartment 15**.

4. A method according to the first part of claim 1, wherein hot exhaust gases, which are produced in **power generator 3**, are supplied by **gas generator 2**, of the third part of claim 1, for accomplishing the process of converting biomass into gases and therefore producing syngas. Non-exhaust power generator can be obtained and gas conversion can be conducted by hot gases containing a proportion of oxygen.

5. The method according to any of claims 1, 2 and 3, wherein hot syngas is removed from chamber 13 for initially cooling it by passage through the **primary treatment tank 9** by two types of liquids; a diluted aqueous acid solution of 3% volume and DMSO as a solvent with 1% mass of chromium chloride as catalyst agent.

6. The method according to any of claims 1, 2, 3 and 5, wherein hot syngas is removed from **chamber** 13 for heating the **primary treatment tank 9** for accomplishing hydrolysis for agricultural waste (converting cellulose materials into glucose) with diluted aqueous acid solution of 3% volume at 180 °C for four to six hours, and converting the produced glucose into fructose by strongly stirring this glucose with DMSO as a solvent (1 liter of DMSO/0.8 kg glucose) with 1% of chromium chloride as a catalyst agent at 90 °C for one hour.

7. The method according to any of claims 1, 2, 3, 5, and 6, wherein the initially cooled syngas is removed from the **primary treatment tank 9** for cooling it in **radiator 4**, to condense tar from the syngas for the purpose of preparing these gases for the compression process or using them as fuel for **power generator 3**.

8. The method according to the last point of the third part of claim 1, wherein the cooled syngas is moved to **compressor 11** for compressing it to 25 bar increasing its temperature to 300 °C and as a result of this compression to prepare it for Fischer-Tropsch process (converting syngas into liquid biodiesel with a catalyst agent). This compressed syngas is moved to **reactor 6** at 25 bar and 300 °C with 75% silicon carbide, 20% cobalt, and 5% calcium as a catalyst agent for producing biodiesel.

9. The method according to any of claims 1, 6 and 8, wherein the produced mixture comprises remaining waste, DMSO, fructose, and catalyst agent and is removed from the **primary treatment tank 9** to **reactor 6** by using **pump 10** for producing HMF by heating this mixture to 120 °C or 250 °C for 2 hours or fifteen minutes, respectively, in **reactor** 6 by compressed and hot syngas.

10. The method according to claim 1, wherein in the LHMF purification process, the previous solution is moved to **purification tank 8** which contains active coal for adsorption of DMSO, and subsequently filtering out LHMF from the bottom of this tank, wherein the correct ratio of active coal to DMSO for adsorption is 10 g : 50 ml.

11. The method according to claim 10, wherein after extraction of DMSO from **purification tank 8** with active coal and adsorbed DMSO, said purification tank can be heated to 192 °C for the evaporation of DMSO and then moving the resulting steam to **radiator 5** to intensify this steam and recapture DMSO for later use.
